(19) Europäisches Patentamt
European Patent Office
Office européen des brevets

(11) **EP 1 997 436 B1**

(12) **EUROPEAN PATENT SPECIFICATION**

(45) Date of publication and mention
of the grant of the patent:
**29.10.2014 Bulletin 2014/44**

(51) Int Cl.:
***A61B 8/08*** (2006.01)

(21) Application number: **07738366.9**

(22) Date of filing: **13.03.2007**

(86) International application number:
**PCT/JP2007/054894**

(87) International publication number:
**WO 2007/108359 (27.09.2007 Gazette 2007/39)**

(54) **ULTRASONOGRAPH**

SONOGRAPH

ULTRASONOGRAPHE

(84) Designated Contracting States:
**DE GB**

(30) Priority: **20.03.2006 JP 2006076855**

(43) Date of publication of application:
**03.12.2008 Bulletin 2008/49**

(73) Proprietor: **Konica Minolta, Inc.**
**Tokyo 100-7015 (JP)**

(72) Inventors:
• **FUKUMOTO, Takenori**
**c/o Matsushita Elec. Ind. Co. Ltd.**
**Shiromi,Chuo-ku, Osaka-shi, Osaka, 540-6207**
**(JP)**
• **HAGIWARA, Hisashi**
**c/o Matsushita Elec. Ind. Co. Ltd.**
**Shiromi, Chuo-ku, Osaka-shi, Osaka, 540-6207**
**(JP)**

• **SUZUKI, Takao**
**c/o Matsushita Elec. Ind. Co. Ldt.**
**Shiromi, Chuo-ku, Osaka-shi, Osaka 540-6207**
**(JP)**

(74) Representative: **Wenning, Ekkehard**
**Henkel, Breuer & Partner**
**Patentanwälte**
**Maximiliansplatz 21**
**80333 München (DE)**

(56) References cited:
EP-A1- 0 958 784          WO-A1-2004/103185
WO-A1-2005/002446     WO-A2-2004/112568
JP-A- 11 318 896          JP-A- 2000 271 117
JP-A- 2001 292 995       JP-A- 2004 344 370
US-A1- 2007 123 777

**Description**

Technical Field of the Invention

The present invention relates to an ultrasonograph for diagnosing a condition of a blood vessel by using an ultrasonic wave.

Background of the Invention

As an example of a method of detecting an Intima-Media Thickness (hereinafter simply referred to as "IMT value") of a carotid artery, i.e., a thickness between an intima and a media of a vascular wall by using an ultrasonic wave, it is well known that the IMT of the carotid artery is measured from a luminance signal indicative of image on an ultrasonic echo coming from the carotid artery and its surrounding tissues on the assumption that the carotid artery has a normal vascular structure (see patent document 1).

The above method however encounters such a problem that, due to the fact that this method relies on an intensity of the ultrasonic echo, a position of a boundary between a blood flow region and an intima of the vascular wall and a position of a boundary between a media and an adventitia of the vascular wall are inaccurately detected when the ultrasonic echo coming from the intima of the vascular wall is low, or when the luminance signal is deteriorated by noises contained in the ultrasonic echo. As a result, the IMT of the carotid artery is inaccurately measured when the ultrasonic echo coming from the intima of the vascular wall is low, or when the luminance signal is deteriorated by noises contained in the ultrasonic echo. Moreover, when the carotid artery has a local pathology such as an atheroma, the position of the boundary between the blood flow region and the intima and the position of the boundary between the media and the adventitia is inaccurately detected on assumption that the carotid artery has a normal vascular structure.

In order to solve this problem, the position of the boundary between the blood flow region and the intima of the vascular wall and the location of the media are detected on the basis of a hardness of tissues calculated from the change of a phase of the ultrasonic echo. The IMT value of the vascular wall is measured from the position of the boundary between the blood flow region and the intima of the vascular wall and the location of the media (e.g. refer to patent document 2). The above-mentioned method however encounters such a problem that, as with the method based on the luminance information, the IMT value of the vascular wall is measured inaccurately when the hardness of tissues has a high level of noises. As a result of the fact that the above-mentioned method focuses only on the hardness of tissues, the position of the boundary between the blood flow region and the intima of the vascular wall and the location of the media, detected on the basis of a hardness of tissues, deviate from those visually recognized by an operator from the luminance information indicative of the intensity of the ultrasonic echo, and tend to be recognized as unnatural and wrong results.

Patent document 1: Japanese Patent Laid-Open Publication H11-318896
Patent document 2: Internationally published 2004/112568 pamphlet

Patent document 2 discloses a device according by the preamble of claim 1.

Disclosure of the Invention

Problem to be solved by the Invention

In order to solve the above problem, the invention is to provide an ultrasonograph which can accurately measure an IMT value of a vascular wall of a blood vessel in a region of interest by combining the intensity of the ultrasonic echo with information from the property of tissues of an object, and automatically detecting the position of the boundary between the blood flow region and the intima and the position of the boundary between the media and the adventitia, which are closely equal to those determined visually by an operator.

Means for solving the Problem

In order to solve the above problem, an ultrasonograph according to the present invention is defined in claim 1. Further embodiments of the invention are defined in the dependent claims.

The ultrasonograph thus constructed can accurately detect the position of the boundary between the blood flow region and the intima and the position of the boundary between the media and the adventitia, without being significantly affected by the change of a luminance value obtained as the intima of the blood vessel, even when the blood vessel has a local pathology such as atheroma. Hence, the position of the boundary between the blood flow region and the intima and the position of the boundary between the media and the adventitia detected and closely equal to those visually recognized from the luminance information obtained on the basis of the intensity of the ultrasonic echo do not recognized as unnatural and wrong results.

The ultrasonograph according to the present disclosure may further comprise an IMT value calculating unit for calculating an IMT value indicative of a thickness of a vascular wall defined by the intima and the media on the basis of position information indicative of the position of the boundary between the blood flow region and the intima and position information indicative of the position of the boundary between the media and the adventitia detected by the boundary detecting unit.

The ultrasonograph thus constructed can accurately measure an IMT value of the blood vessel.

The blood vessel has a vascular wall defining a blood flow region through which blood flows, the blood flow region being defined by a front vascular wall close to the ultrasonic signal emitting unit and a back vascular wall far from the ultrasonic signal emitting unit in sectional view. The ultrasonograph according to the present disclosure may further comprise a region determining unit for determining a region of interest covering at least one of the front vascular wall and the back vascular wall. The amplitude information processing unit may be adapted to process the amplitude information of the ultrasonic echo from the region of interest determined by the region determining unit. The phase processing unit may be adapted to process the phase information of the ultrasonic echo from the region of interest determined by the region determining unit.

The ultrasonograph thus constructed can detect the position of the boundary between the blood flow region and the intima and the position of the boundary between the media and the adventitia.

In the ultrasonograph according to the present disclosure, the boundary detecting unit may be adapted to detect, on the basis of one heart cycle, the position of the boundary between the blood flow region and the intima and the position of the boundary between the media and the adventitia from the processing result outputted from the amplitude information processing unit and the processing result outputted from the phase information processing unit.

The ultrasonograph thus constructed can more accurately detect the position of the boundary between the blood flow region and the intima and the position of the boundary between the media and the adventitia.

In the ultrasonograph according to the present disclosure, the ultrasonic signal emitting unit may be adapted to emit at least one ultrasonic pulse signal in a direction toward at least one point on a longitudinal axis of the blood vessel. The amplitude information processing unit may be adapted to perform processing on the basis of an ultrasonic echo coming from the direction toward the point on the longitudinal axis of the blood vessel. The phase information processing unit may be adapted to perform processing on the basis of the ultrasonic echo coming from the direction toward the point on the longitudinal axis of the blood vessel. The boundary detecting unit may be adapted to detect the position of the boundary between the blood flow region and the intima and the position of the boundary between the media and the adventitia on the basis of at least one processing result outputted from the amplitude information processing unit and at least one processing result outputted from the phase information processing unit.

The ultrasonograph thus constructed can accurately detect a boundary between a blood flow region and an intima along the longitudinal axis of the blood vessel, and a position of the boundary between the media and the adventitia.

The ultrasonograph according to the present disclosure, may further comprise a display unit for displaying, as an image, the amplitude information processed by the amplitude information processing unit, the phase information processed by the phase information processing unit, and the position information representing the the position of the boundary between the blood flow region and the intima and the position information indicative of the position of the boundary between the media and the adventitia detected by the boundary detecting unit.

The ultrasonograph thus constructed can allow a user to visually recognize the position of the boundary between the blood flow region and the intima and the position of the boundary between the media and the adventitia.

In the ultrasonograph according to the present disclosure one of processing results outputted from the amplitude information processing unit may indicate amplitude of an ultrasonic echo coming from a depth direction of the object.

The ultrasonograph thus constructed can more accurately detect the the position of the boundary between the blood flow region and the intima and the position of the boundary between the media and the adventitia.

In the ultrasonograph according to the present disclosure, one of processing results outputted from the amplitude information processing unit may indicate the rate of change of an ultrasonic echo coming from a depth direction of the object.

The ultrasonograph thus constructed can more accurately detect the the position of the boundary between the blood flow region and the intima and the position of the boundary between the media and the adventitia.

In the ultrasonograph according to the present disclosure, one of processing results outputted from the amplitude information processing unit may indicate a hardness of tissues of the object calculated in a depth direction of the object, from the phase information of the ultrasonic echo.

The ultrasonograph thus constructed can more accurately detect the position of the boundary between the media and the adventitia.

In the ultrasonograph according to the present disclosure, one of processing results outputted from the amplitude information processing unit may indicate a strain of tissues of the object calculated, in a depth direction of the object, from the phase information of the ultrasonic echo on the basis of one heart cycle.

The ultrasonograph thus constructed can more accurately detect the position of the boundary between the media and the adventitia.

In the ultrasonograph according to the present disclosure, one of processing results outputted from the amplitude information processing unit may indicate a thickness of tissues of the object calculated, in a depth direction of the object, from the phase information of the ultrasonic echo.

The ultrasonograph thus constructed can more accurately detect the position of the boundary between the media and the adventitia.

In the ultrasonograph according to the present disclosure, one of processing results outputted from the amplitude information processing unit may indicate a moving velocity of tissues of the object calculated, in a depth direction of the object, from the phase information of the ultrasonic echo on the basis of one heart cycle.

The ultrasonograph thus constructed can more accurately detect the position of the boundary between the media and the adventitia.

In the ultrasonograph according to the present disclosure, the boundary detecting unit may be adapted to determine a region of detection in a depth direction of the object on the basis of at least one processing result outputted from the amplitude information processing unit, and to detect the position of the boundary between the blood flow region and the intima on the basis of at least one processing result outputted from the phase information processing unit in the region of detection.

The ultrasonograph thus constructed can more accurately detect the position of the boundary between the blood flow region and the intima and the position of the boundary between the media and the adventitia.

In the ultrasonograph according to the present disclosure, the processing result outputted from the amplitude information processing unit may indicate an intensity of the ultrasonic echo. The processing result outputted from the phase information processing unit may indicate a hardness of tissues of the object calculated, in the depth direction of the object, from the phase information of the ultrasonic echo.

The ultrasonograph thus constructed can more accurately detect the position of the boundary between the media and the adventitia.

In the ultrasonograph according to the present disclosure, the boundary detecting unit may be adapted to determine a region of detection in a depth direction of the object on the basis of at least one processing result outputted from the amplitude information processing unit, and to detect the position of the boundary between the media and the adventitia on the basis of at least one processing result outputted from the phase information processing unit in the region of detection.

The ultrasonograph thus constructed can more accurately detect the position of the boundary between the media and the adventitia.

In the ultrasonograph according to the present disclosure, at least one processing result outputted from the amplitude information processing unit may indicate an intensity of an ultrasonic echo coming from a depth direction of the object and the rate of change of the intensity of the ultrasonic echo.

The ultrasonograph thus constructed can more accurately detect the position of the boundary between the media and the adventitia.

In the ultrasonograph according to the present disclosure, at least one processing result outputted from the amplitude information processing unit may be filtered in a depth direction of the object, and then outputted to the boundary detecting unit.

The ultrasonograph thus constructed can more accurately detect the position of the boundary between the blood flow region and the intima and the position of the boundary between the media and the adventitia without being significantly affected by noises contained in the ultrasonic echo.

In the ultrasonograph according to the present disclosure, at least one processing result outputted from the amplitude information processing unit may be filtered in a depth direction of the object and in a longitudinal direction of the blood vessel, and then outputted to the boundary detecting unit.

The ultrasonograph thus constructed can more accurately detect the position of the boundary between the blood flow region and the intima and the position of the boundary between the media and the adventitia without being significantly affected by noises contained in the amplitude information obtained from the ultrasonic echo.

In the ultrasonograph according to the present disclosure, at least one processing result outputted from the phase information processing unit may be filtered in a depth direction of the object, and then outputted to the boundary detecting unit.

The ultrasonograph thus constructed can more accurately detect the position of the boundary between the blood flow region and the intima and the position of the boundary between the media and the adventitia without being significantly affected by noises contained in the phase information obtained from the ultrasonic echo.

In the ultrasonograph according to the present disclosure, at least one processing result outputted from the phase information processing unit may be filtered in a depth direction of the object and in a longitudinal direction of the blood vessel, and then outputted to the boundary detecting unit.

The ultrasonograph thus constructed can more accurately detect the position of the boundary between the blood flow region and the intima and the position of the boundary between the media and the adventitia without being significantly affected by noises contained in the phase information obtained from the ultrasonic echo.

In the ultrasonograph according to the present disclosure, the position information indicative of the position of the boundary between the blood flow region and the intima and the position information indicative of the position of the boundary between the media and the adventitia detected by the boundary detecting unit may be filtered in a longitudinal direction of the blood vessel, and then outputted to the IMT value calculating unit.

The ultrasonograph thus constructed can accurately measure an IMT value of a blood vessel, avoid a significant deviation of the detected boundary from a boundary determined visually by an operator based on the luminance information obtained from an intensity of the ultrasonic echo, and eliminate the unnatural and wrong results due to the above deviation of these boundary positions.

In the ultrasonograph according to the present disclosure, the position information indicative of the position of the boundary between the blood flow region and the intima and the position information indicative of the position of the boundary between the media and the adventitia detected by the boundary detecting unit may be filtered in a longitudinal direction of the blood vessel, and then outputted to the display unit

The ultrasonograph thus constructed can accurately measure an IMT value of a blood vessel, avoid a significant deviation of the detected boundary from a boundary determined visually by an operator based on the luminance information obtained from an intensity of the ultrasonic echo, and eliminate the unnatural and wrong results due to the above deviation of these boundaries.

Advantageous Effect of the Invention

From the above-mentioned characteristics, it is understood that the ultrasonograph according to the present invention can accurately detect a boundary between a blood flow region and a vascular wall, without being affected by the change of a luminance value obtained as an intima of the vascular wall, even when the blood vessel has a local pathology such as atheroma. Furthermore, the position of the boundary between the blood flow region and the intima and the position of the boundary between the media and the adventitia detected and closely equal to those visually recognized from the luminance information obtained on the basis of the intensity of the ultrasonic echo do not recognized as unnatural and wrong results. Consequently, the ultrasonograph according to the present invention can accurately measure an IMT value of a vascular wall of a blood vessel in a region of interest by combining the intensity of the ultrasonic echo with information from the property of tissues of an object, and automatically detecting the position of the boundary between the blood flow region and the intima and the position of the boundary between the media and the adventitia, which are closely equal to those determined visually by an operator.

Brief Description of the Drawings

FIG. 1 is a block diagram showing a general construction of an ultrasonograph according to the first embodiment of the present invention.

FIG. 2 is a schematic diagram for explaining an operation of the ultrasonograph according to the first embodiment of the present invention.

FIG. 3 is a schematic diagram for explaining an operation of the ultrasonograph according to the first embodiment of the present invention.

FIG. 4 is a block diagram showing a general construction of an ultrasonograph according to the second embodiment of the present invention.

FIG. 5 is a schematic diagram for explaining an example of operations of the first and second filter units forming part of the ultrasonograph according to the second embodiment of the present invention.

FIG. 6 is a schematic diagram for explaining another example of operations of the first and second filter units forming part of the ultrasonograph according to the second embodiment of the present invention.

FIG. 7 is a schematic diagram for explaining an example of an operation of the third filter unit of the ultrasonograph according to the second embodiment of the present invention.

Explanation of the Reference Numerals

**1:** transmitting unit
**2:** receiving unit (ultrasonic echo receiving unit)
**3:** delay/synthesis unit
**4:** B-mode processing unit
**5:** amplitude information processing unit
**6:** phase information processing unit
**7:** boundary detecting unit
**8:** region determining unit
**9:** IMT value calculating unit
**10:** image synthesizing unit
**11:** display unit (display means)
**20:** skin surface of an object to be inspected
**25:** first filter unit
**26:** second filter unit
**27:** third filter unit
**28:** filter control unit
**30:** blood vessel
**40:** region of interest
**51, 52:** processing result of amplitude information
**61, 62:** processing result of phase information
**71:** position information indicative of the position of the boundary between the blood flow region and the intima
**72:** position information indicative of the position of the boundary between media and adventitia
**73:** threshold
**74:** offset
**101:** ultrasonic probe (ultrasonic signal emitting unit)
**120, 121:** region in which detection
**301:** adventitia
**302:** internal media
**304:** blood flow region
**305:** first vascular wall
**306:** atheroma
**310:** boundary between blood flow region and intima
**311:** second vascular wall
**320:** boundary between media and adventitia

Preferred Embodiments of the Invention

Preferred embodiments of the ultrasonograph according to the present invention will be described hereinafter with reference to the drawings.

(First embodiment)

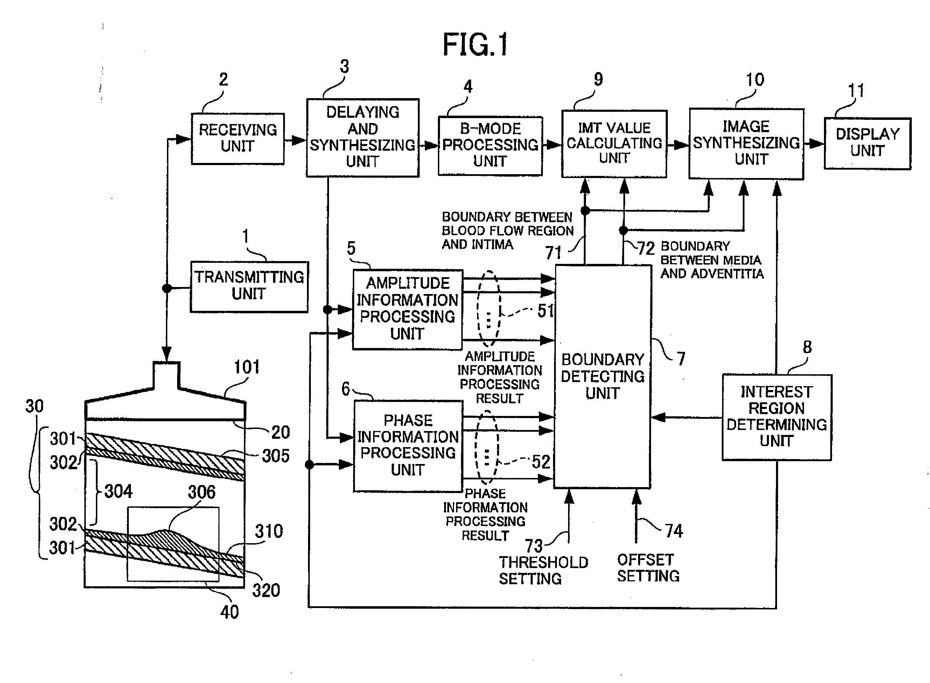

FIG. **1** is a block diagram showing a general construction of an ultrasonograph according to the first embodiment of the present invention. As shown in FIG. **1,** the ultrasonograph according to the first embodiment comprises a transmitting unit **1** for generating at least one ultrasonic pulse signal, an ultrasonic probe **101** for emitting the ultrasonic pulse signal generated by the transmitting unit **1** in a direction from a skin surface **20** of an object such as a biological body to a blood vessel **30** inside the object, and receiving an ultrasonic echo from the blood vessel **30** as a reflection of the ultrasonic pulse signal from the blood vessel **30**, a receiving unit **2** for converting the ultrasonic echo received by the ultrasonic probe **101** into an ultrasonic echo, a delay/synthesis unit **3** which, if needed, delays and synthesizes the components of the ultrasonic echo produced by the receiving unit **2,** a brightness modulation mode processing unit **4** (hereinafter simply referred to as "B-mode processing unit") for producing sectional image information on the blood vessel **30** from the ultrasonic echo outputted from the delay/synthesis unit **3,** an amplitude information processing unit **5** for applying at least one processing to amplitude information indicative of an amplitude of the ultrasonic echo outputted from the delay/ synthesis unit **3,** and outputting a processing result, and a phase information processing unit **6** for applying at least one processing to phase information indicative of a phase of the ultrasonic echo outputted from the delay/synthesis unit **3,** and outputting a processing result. The ultrasonograph according to the first embodiment further comprises a boundary detecting unit **7** for detecting, on the basis of the processing result outputted from the amplitude information processing unit **5** and the processing result outputted from the phase information processing unit **6,** a boundary **310** between a blood flow region and an intima and a boundary **320** between a media and an adventitia **301,** outputting position information **71** indicative of the position of the boundary **310** between the blood flow region and the intima and position information **72** indicative of the position of the boundary **320** between the media and the adventitia **301,** and producing color display image information indicating a sectional image of a blood vessel mapped to a two-dimensional plane, a region determining unit 8 for determining a region of interest (ROI) **40** along a depth direction, i.e., a direction from the skin surface **20** of the object to the blood vessel **30** inside the object to ensure that the amplitude information and the phase information are computed in the region of interest **40,** an IMT value calculating unit **9** for calculating an IMT value from the position information **71** indicative of the position of the boundary **310** between the blood flow region and the intima and the position information **72** indicative of the position of the boundary **320** between the media and the adventitia **301,** an image synthesizing unit **10** for combining the image information produced by the B-mode processing unit **4** with the image information produced by the boundary detecting unit **7,** and a display unit **11** for displaying an image on the basis of the combined image information produced by the image synthesizing unit **10.** The ultrasonic probe **101** functions as an ultrasonic signal emitting unit. The receiving unit **2** functions as an ultrasonic echo receiving unit.

In this embodiment, the blood vessel **30** has a vascular wall defining a blood flow region (passageway) **304** through which blood flows. In a longitudinal sectional view, the vascular wall of the blood vessel **30** is defined by a first vascular wall **305** closer to the ultrasonic probe **101,** and a second vascular wall **311** far from the ultrasonic probe **101.** In this case, the blood vessel **30** defined by the first and second vascular walls **305** and **311** has a local pathological change called "atheroma" **306.** The region determining unit **8** is adapted to determine a region of interest **40** along a depth direction, i.e., a direction from the skin surface **20** of the object to the blood vessel **30** inside the object to ensure that the amplitude information and the phase information are computed in the region of interest **40** under the condition that both or either the first vascular wall **305** or the second vascular wall **311** is in the region of interest **40.** However, the region of interest **40** may be determined by the region determining unit **8** in cooperation with other devices, or may be determined by an operator's manipulation.

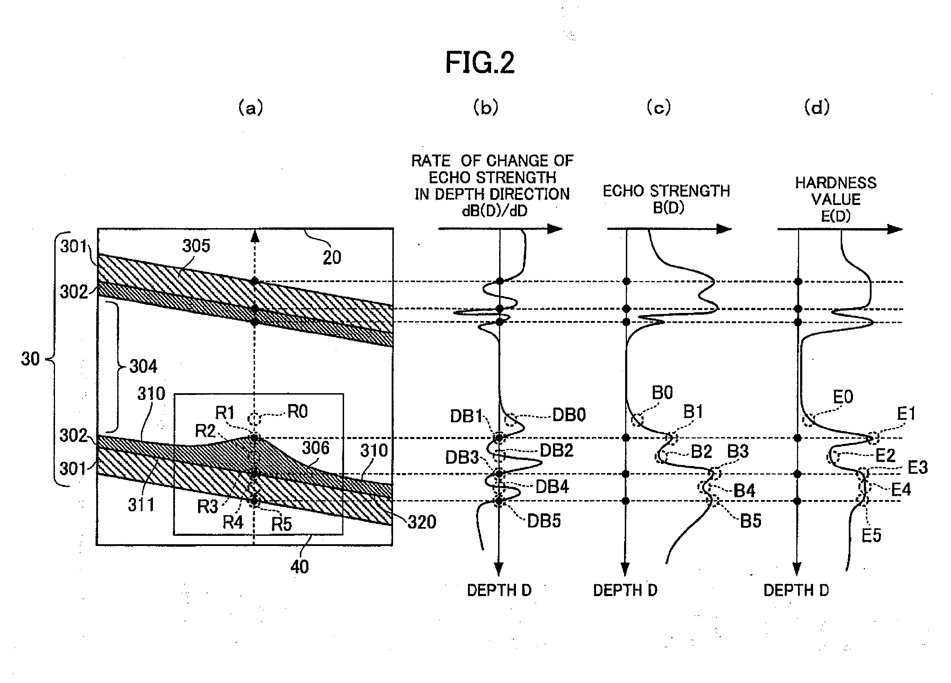

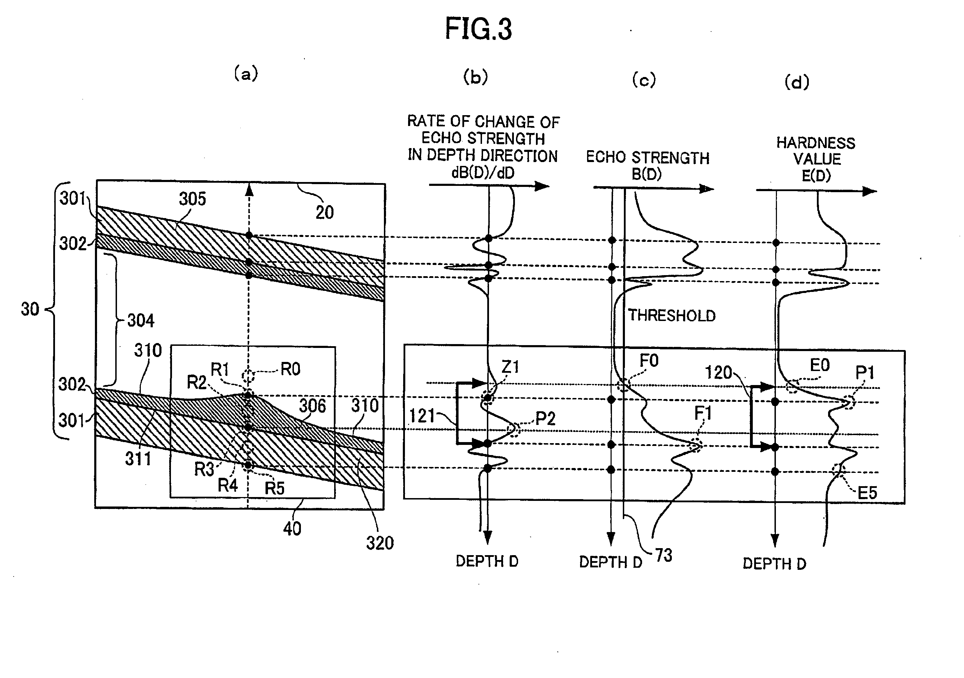

The basic operation of the ultrasonograph according to the first embodiment will be explained hereinafter with reference to FIGS. **2** and **3.**

FIG. **2** is a schematic diagram for explaining the position of the boundary **310** between the blood flow region and the intima and the boundary **320** between the media and the adventitia **301.** In this case, the boundary **310** between the blood flow region and the intima and the boundary **320** between the media and the adventitia **301** are detected by the boundary detecting unit **7** from an intensity B(D) and the rate of change of the ultrasonic echo in the depth direction dB(D)/dD at a depth **D** which are computed by the amplitude information processing unit **5** on the basis of the ultrasonic echo received from the blood vessel **30** as a reflection of one emitted ultrasonic pulse signal from the blood vessel **30,** and a hardness E(D) of tissues at a depth **D** which is computed by the phase information processing unit **6** on the basis of the phase of the ultrasonic echo.

The following description is directed to points **R0** to **R5** defined on a scanning line representing a path of an ultrasonic pulse signal. As shown in FIG. **2(a),** the point **R0** is within the blood flow region **304** of the blood vessel **30.** The point **R1** is on a boundary between the second vascular wall **311** and the blood flow region **304.** The point **R2** is on an atheroma 306 formed in the second vascular wall **311.** The point **R3** is on a boundary between a media contained in an internal media **302** and an adventitia **301.** The point **R5** is on a boundary between an adventitia **301** and outside tissues of the blood vessel **30.** As shown in FIG. **2(c),** it is ideal that the intensity B(D) of the ultrasonic echo has two distinguishable peaks **B1** and **B2,** the peak **B1** corresponding to the point **R1** indicative of the position of the boundary **310** between the blood flow region and the intima, the peak **B3** corresponding to the point **R3** indicative of the position of the boundary **320** between the media and the adventitia **301.** Then, the location of the peaks can be clearly detected if the blood vessel is normal and if the ultrasonic echo measurement is performed accurately. However, in case of measuring the blood vessel having an atheroma to be diagnosed in detail, or in case of measuring a general blood vessel, the intensity of the ultrasonic echo may lack the above recognizable pair of peaks due to the fact that the intensity of the ultrasonic echo may moderately changed from **B0** that corresponds to a blood flow region **304** through **B1** which corresponds to the point **R1** indicative of the position of the boundary **310** between the blood flow region and the intima, to **B2** which corresponds to **R2** within the atheroma, or it may contain noises. Therefore, it is impossible to accurately detect the boundary **310** between the blood flow region and the intima and the boundary **320** between the media and the adventitia **301.**

On the other hand, as shown in FIG. **2(d),** due to the fact that the hardness E(D) of tissues has a peak value **E1** which corresponds to the point **R1** defined indicative of the position of the boundary **310** between the blood flow region and the intima, and which is significantly more clear than that of the intensity B(D) of the ultrasonic echo, it is possible to accurately detect the boundary **310** between the blood flow region and the intima in comparison with the case of using the intensity B(D) of the ultrasonic echo. However, by taking the method to detect the maximum peak value within the region of interest **40,** a separate peak value may result independent of a boundary **310** between the blood flow region and the intima due to the effect of noise occurring as the result of computation of the measured phase of the ultrasonic echo or the hardness E(D) of tissues. This causes a problem of generating a false peak value within the region of interest **40.** Also, the hardness E(D) of tissues lacks a clear key property necessary to detect the boundary **320** between the media and the adventitia **301** though the hardness E(D) of tissues has a property that the boundary **310** between the blood flow region and the intima is detectable.

In order to solve the above problems, the ultrasonograph according to the first embodiment restricts, on the basis of the intensity B(D) of the ultrasonic echo, a region in which the boundary **310** between the blood flow region and the intima is detected on the basis of the hardness E(D) of tissues by the boundary detecting unit **7.**

The summary of this process will be explained with reference to FIG. **3.** The diagram of FIG. **3,** similar to that of FIG. **2,** shows an intensity B(D) of an ultrasonic echo from tissues, the rate of change of the intensity of the ultrasonic echo dB(D) /dD in depth direction at the depth **D** computed by the amplitude information processing unit **5,** and the hardness E(D) of tissues at the depth **D** computed by the phase information processing unit **6** from the phase of the ultrasonic echo. Here, a region in which the intensity B(D) of the ultrasonic echo, the rate of change of the intensity of the ultrasonic echo dB(D)/dD, and the hardness E(D) of tissues are computed, corresponding to the region of interest **40**, is circumscribed and restricted by a solid line. In this embodiment, the important point is to determine a detection region where the amplitude information processing unit **5** and the phase information processing unit **6** detect within the region of interest **40.**

Specifically, a region of detection **120,** in which the hardness E(D) of tissues in the depth **D** is obtained by the phase information processing unit **6,** is determined through the following process. In FIG. **3(c),** a point **F0** is determined as a point at which the intensity B(D) of the ultrasonic echo exceeds a predetermined threshold level **73** when searching from the skin surface within a region circumscribed by a solid line corresponding to the region of interest **40.** Next, a position **F1** is determined as a position at which the intensity B(D) reaches a maximum value when searching towards the direction away from the skin surface. Then, a region in FIG. **3(d)** corresponding to a region between **F0** and **F1** indicated in FIG. **3 (c)** is defined as the region of detection **120**, and a position at which the hardness E(D) of the tissue becomes maximum in the region of detection **120** is defined as **P1** which corresponds to the point **R1** on a boundary **310** between the blood flow region and the intima. As shown in FIG. **1,** the above-mentioned threshold **73** can be prescribed from outside.

In this embodiment, when detecting a boundary **310** between the blood flow region and the intima, the hardness E(D) of tissues is used as the processing result outputted from the phase information processing unit **6.** The hardness E(D) of tissues is along the body surface to the depth direction computed based on the phase of the ultrasonic echo. However, the invention is not limited to this value. As the processing result outputted from the phase information processing unit **6,** any results with a key property that can be used to detect a boundary **310** between the blood flow region and the intima may be used. For example, the followings may be used as the processing result outputted from the phase information processing unit **6.** Namely, these are the strain of the tissues of the object along the body surface to the depth direction based on the rate of change in time for one heart cycle, the thickness of the tissues of the object along the body surface to the depth direction, and the moving velocity of the tissues of the object along the body surface to the depth direction based on the rate of change in time for one heart cycle.

In the above explanation, when detecting a boundary **310** between the blood flow region and the intima, the property of the hardness E(D) of tissues at the depth **D** based on the phase of the ultrasonic echo computed by the phase information processing unit is paid an attention. However, the present invention is not limited to the use of this value. For instance, any value which clearly reveals the characteristic of the tissue of a blood flow different from that of a vascular wall can be used, such as the tissue thickness value, the strain value, or the high frequency component of the velocity. More specifically, any methods which can accurately detect a boundary **310** between the blood flow region and the intima may be used. For example, these methods are: measuring the strain of tissues of the object along a body surface to a depth direction based on the rate of change in time for one heart cycle, measuring the thickness of tissues of the object to be measured along a body surface to the depth direction, and measuring the velocity of the motion of tissues of the object along a body surface to the depth direction based on the rate of change in time for one heart cycle. For instance, in a blood flow region, hardness of tissues is low, but a high frequency component of the velocity of tissues motion is large. On the other hand, in a vascular wall region, hardness of tissues is high, but a high frequency component of the velocity of tissues motion is small. By focusing on such a property, a restriction on the region of detection may be limited according to the high frequency component of the velocity of tissues motion in one heart cycle obtained from the phase information processing unit 6, and within such an region of detection, the locations at which the hardness of tissues takes the maximum value may be detected to be the position information indicative of the position of the boundary between the blood flow region and the intima.

When detecting the boundary **320** between the media and the adventitia **301** as well as the boundary **310** between the blood flow region and the intima, it would be ideal if a clear peak is identified from measured values. Thus, by focusing only on the intensity B(D) of the ultrasonic echo, the simplest method is to take a location at which the intensity B(D) of the ultrasonic echo takes a peak value as the boundary **320** between the media and the adventitia **301,** separated from those locations which has already been determined to be the boundary **310** between the blood flow region and the intima. However as stated above, in real measurements, there are many cases in which the boundary **310** between the blood flow region and the intima is unclear. As shown in FIG. **2(c),** the intensity B(D) of the ultrasonic echo may have two distinguishable peaks in the ideal boundary 310 between the blood flow region and the intima and the boundary **320** between the media and the adventitia **301,** or noises may generate a false peak in B(D). Therefore, it is difficult to accurately detect the boundary **320** between the media and the adventitia **301.** Alternatively, it is possible to consider a method to detect the boundary based on the positions at which the rate of change of the intensity B(D) of the ultrasonic echo dB(D)/dD in the depth direction crosses a zero or takes a peak value. However, for the same reasons described above, if only one of B (D) or dB(D)/dD is used, the position of the boundary **320** between the media and the adventitia **301** becomes difficult. Also, the hardness E(D) of tissues does not have clear property that is able to detect the boundary **320** between the media and the adventitia **301.**

The ultrasonograph according to the first embodiment detects the position at which the rate of change of the intensity B(D) of the ultrasonic echo dB(D)/dD in a depth direction takes a positive maximum peak value within a region of detection **121** restrictively defined according to the intensity B(D) of the ultrasonic echo, and detects the boundary **320** between the media and the adventitia. This detection enables the boundary **320** between the media and the adventitia **301** to be more accurately detected. This is summarized according to FIG. 3.

More specifically, the region of detection 121 for the rate of change dB(D)/dD along the depth direction of the intensity B(D) of the ultrasonic echo obtained by the amplitude information processing unit **5** is determined by the following steps. In the region of detection **120** indicated in FIG. **3(d)** which was determined in order to detect the boundary **310** between the blood flow region and the intima, that is, a region between **F0** and **F1,** the area that corresponds to FIG. **3(b)** is prescribed as the region of detection **121.** Then, within this region of detection **121,** a position at which the rate of change of the intensity B(D) of the ultrasonic echo dB(D)/dD in a depth direction takes the last maximum value is searched in the order from the point **F0,** and this point of taking the last maximum value is set as the point **P2** corresponding to the point **R3** indicative of the position of the boundary **320** between the media and the adventitia **301.** This process enables the boundary **310** between the blood flow region and the intima and the boundary **320** between the media and the adventitia **301** to be accurately be detected, even if the intensity B(D) of the ultrasonic echo does not have a distinguishable peak in the boundary **310** between the blood flow region and the intima or the boundary **320** between the media and the adventitia **301.**

In this explanation, the region of detection is prescribed by focusing on the property of the intensity B(D) of the ultrasonic echo when the boundary detecting unit 7 detects the boundary **320** between the media and the adventitia **301.** The invention is not limited to this prescription. Any determining methods that can accurately measure the boundary **320** between the media and the adventitia **301** may be obviously applied to this prescription. For example, the inflection point of the intensity B(D) of the ultrasonic echo may also be used to determine the region of detection, rather than using the intensity B(D) of the ultrasonic echo or its rate of change in a depth direction.

The IMT value calculating unit **9** computes an IMT value from the position information **71** indicative of the position of the boundary **310** between the blood flow region and the intima and the position information **72** indicative of the position of the boundary **320** between the media and the adventitia **301,** which are detected by the boundary detecting unit 7. Then, an IMT value calculating unit **9** sends the IMT value to the image synthesizing unit **10.** The B-mode processing unit **4** produces the image information representing the cross section of the blood vessel **30** based on the ultrasonic echo which passes through the delay/synthesis unit **3,** and submits this image information to the image synthesizing unit **10.** The image synthesizing unit **10** then combines the image information provided by the B-mode processing unit **4** with the result provided by the boundary detecting unit **7.** Then, the display unit **11** including monitors and other parts displays the image based on the image data combined by the image synthesizing unit **10.** When detecting a misalignment between the B-mode image displayed on the display unit **11** and the position of the boundary detected, a predetermined fixed value may be added to or subtracted from the position information **71** indicative of the position of the boundary between the blood flow region and the intima and the position information **72** indicative of the position of the boundary between the media and the adventitia inputted from the boundary detecting unit **7** in order to eliminate the misalignment. More specifically, as shown in FIG. **1,** the offset value **74** and the threshold **73** may be set from the outside. In the ultrasonograph according to the embodiment, this setting is performed in the boundary detecting unit **7.** However, the invention is not restricted to this setting. Further, the image synthesizing unit **10** displays an IMT value calculated by the IMT value calculating unit **9** if necessary, but the invention is not restricted to such dsiplaying. It may suffice for the display unit **11** to display a boundary that is close to the case in which an operator visually determines such boundary.

Also, due to the fact that the relative position of the ultrasonic probe **101** and the above-mentioned boundaries change in one heart cycle (interval between two R waves), an IMT value varies depending on the timing of calculating an IMT value. However, the IMT value is normally measured during the diastolic when a vascular wall is not contracting. Hence, the timing at which to calculate an IMT value can be determined during the neighborhood of the diastolic in one heart cycle during which an IMT value takes a maximum value. For example, the boundary can be detected very second between one R wave to the next R wave and the distance between the position of the boundary between the blood flow region and the intima and the position of the boundary between the media and the adventitia is calculated occasionally, and the maximum value can be set to be a an IMT value. But, the invention does not restrict the determining method to just this example.

As explained above, the ultrasonograph according to the second embodiment comprises an ultrasonic probe **101** for emitting at leass one ultrasonic pulse signal in a direction from a skin surface of an object toward a blood vessel inside the object, and receiving an ultrasonic echo from the blood vessel as a reflection of the ultrasonic pulse signal from the blood vessel, a receiving unit **2** for converting the ultrasonic echo received by the ultrasonic probe **101** into an ultrasonic echo, an amplitude information processing unit **5** for processing amplitude information indicative of an amplitude of the ultrasonic echo coming from a direction intersecting a longitudinal axis of the blood vessel, a phase information processing unit **6** for processing phase information indicative of a phase of the ultrasonic echo coming from a direction intersecting a longitudinal axis of the blood vessel, a boundary detecting unit **7** for detecting, on the basis of at least one processing result outputted from the amplitude information processing unit **5** and at least one processing result outputted from the phase information processing unit **6,** a boundary between a blood flow region and an intima and a boundary between a media and an adventitia **301,** and an IMT value calculating unit **9** for calculating an IMT value from the position information indicative of the position of the boundary between the blood flow region and the intima and the position information indicative of the position of the boundary between the media and the adventitia **301** detected by the boundary detecting unit **7.** Therefore, the ultrasonograph according to the present invention is able to accurately detect the position information indicative of the position of the boundary between the blood flow region and the intima and the position information indicative of the position of the boundary between the media and the adventitia without being significantly affected by the change of a luminance value obtained as an intima of the vascular wall even when the blood vessel has a local pathology such as for example atheroma. Moreover, the configuration in the embodiment enables the detected boundary position not to significantly deviate from the position of each boundary visually recognized from the luminance information obtained on the basis of the intensity of the ultrasonic echo, and do not recognized as unnatural and wrong results. Furthermore, the ultrasonograph according to the present invention can accurately measure an IMT value of a vascular wall of a blood vessel in a region of interest.

(Second Embodiment)

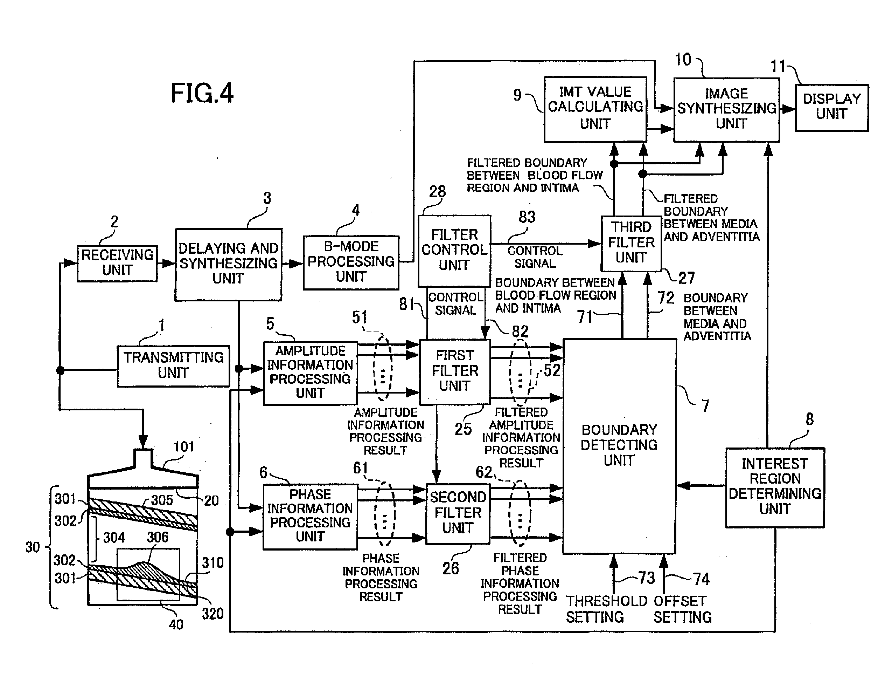

FIG. **4** is a block diagram showing a general construction of an ultrasonograph according to the second embodiment of the present invention. In FIG. **4,** elements of the ultrasonograph according to the second embodiment the same as those of the ultrasonograph according to the first embodiment bear the same reference numerals as those of the ultrasonograph according to the first embodiment, and does not explained hereinafter. A method of obtaining position information **71** indicative of the position of the boundary between the blood flow region and the intima and position information **72** indicative of the position of the between the media and the adventitia is the same as a method explained in the first embodiment, and does not explained hereinafter.

As shown in FIG. **4,** the ultrasonograph according to the second embodiment comprises, in addition to the constitution of the ultrasonograph according to the first embodiment, a first filter unit **25** for filtering processing results outputted from the amplitude information processing unit **5,** and outputting this filtered results to the boundary detecting unit **7,** a second filter unit **26** for filtering processing results outputted from the phase information processing unit **6,** and outputting this filtered results to the boundary detecting unit **7,** a third filter unit **27** for filtering the processing results outputted from the boundary detecting unit **7,** and outputting this filtered results to the IMT value calculating unit **9** as well as to the image synthesizing unit **10,** and a filter control unit **28** for controlling the first filter unit **25,** the second filter unit **26,** and the third filter unit **27.**

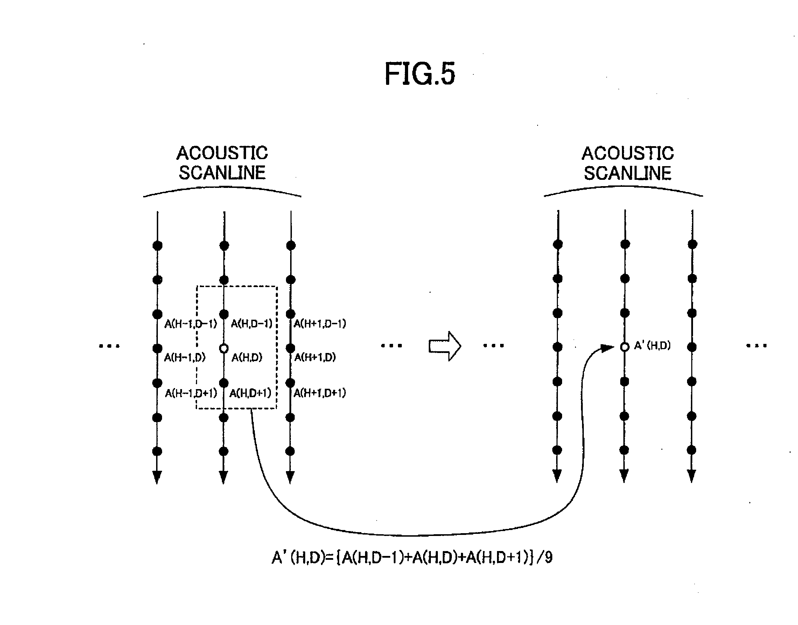

The operation of the ultrasonograph according to the second embodiment will be described hereinafter with reference to FIGS. **5** to **7.** The operation of the ultrasonograph according to the second embodiment is substantially the same as that of the ultrasonograph according to the first embodiment with the exception of operations of the first filter unit **25,** the second filter unit **26,** the third filter unit **27,** and the filter control unit **28.** Therefore, the operation of the ultrasonograph according to the second embodiment the same as that of the ultrasonograph according to the first embodiment will be not described hereinafter.

The processing results outputted from the amplitude information processing unit **5** and the processing result outputted from the phase information processing unit **6** are filtered in the depth direction from the body surface by the first filter unit **25** and the second filter unit **26,** and then outputted to the boundary detecting unit **7.**

The following description is directed to an example of a filtering processing to be executed by the first filter unit **25.** In FIG. **5,** the position of the boundary K(H) is on an acoustic scanline H showing a path of an ultrasonic pulse signal. One of the filtered results outputted to the IMT value calculating unit **9** from the first filter unit **25** is represented by A'(H, D), obtained through step of filtering the amplitude information processing result **51** in a depth direction, and expressed as the following formula.

$$A'(H, D) = \{A(H, D-1) + A(H, D) + A(H, D+1)\} / 3$$

This example is directed to the simplest weighted mean average filter applied to the first filter unit **25** in which the amplitude information processing result **51** is filterd. Similarly, the phase information processing result **61** is also filtered by the simplest weighted mean average filter applied to the second filter unit **26,** and outputted to the boundary detecting unit **7.**

In this case, the first filter unit **25** performs the weighted mean average of three amplitude information processing results A(H, D-1), A(H, D), and A(H, D+1). The amplitude information processing result A(H, D) corresponds to a target point, while the amplitude information processing results A(H, D-1) and A(H, D+1) correspond to two neighboring points. The second filter unit **26** performs the weighted mean average of three phase information processing results. However, the present invention is not limited to this case. For example, the number of data needed for the simplest weighted mean average is not limited. The first and second filter units **25** and **26** may be constituted by conventional two dimentional FIR filters, two dimentional non-linear filters, or the like. The first and second filter units **25** and **26** may be operable to remive noises from the amplitude information processing result **51** and the phase information processing result **61**. It is obvious that the filter applied to the amplitude information processing result **51** may be different in type or in characteristics from that applied to the phase information processing result **61**.

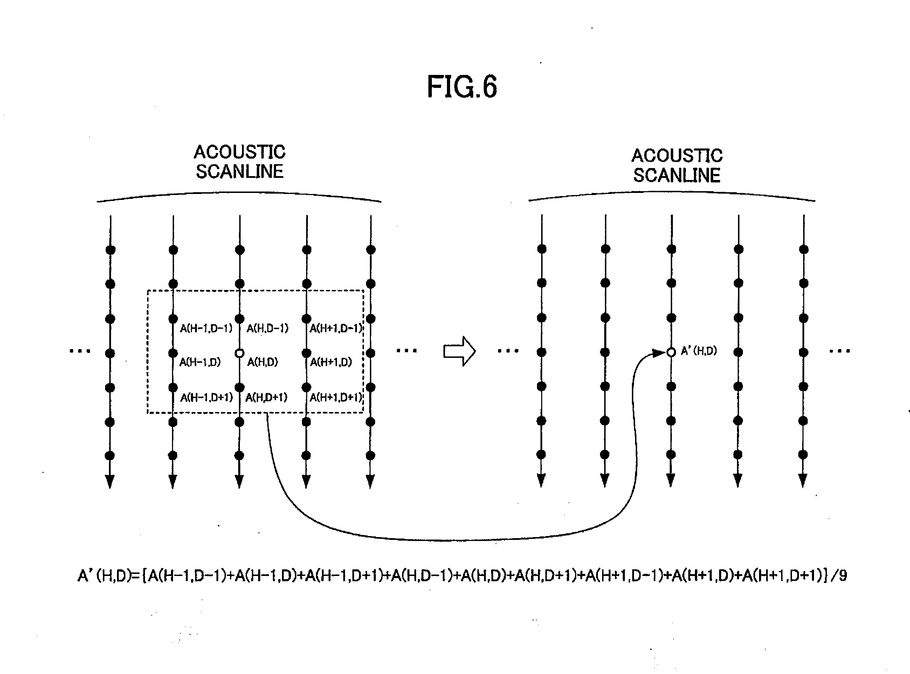

FIG. **6** is a schematic diagram for explaining another example of operations of the first and second filter units forming part of the ultrasonograph according to the second embodiment of the present invention. In FIG. **6,** the amplitude information processing result **51** corresponding to a depth **D** on a particular acoustic scanline H is represented by a function A(H, D), and one filtered result to be outputted from the first filter unit **25** to the boundary detecting unit **7** is represented by a function A"(H, D). When the amplitude information processing result **51** is filtered along a direction of the acoustic scanline H and a depth direction from the skin surface, the filtered result A"(H, D) can be expressed by the following expression.

$$A''(H, D) = \{A(H-1, D-1) + A(H-1, D) + A(H-1, D+1) + A(H, D-1)$$
$$+ A(H, D) + A(H, D+1) + A(H+1, D-1) + A(H+1, D) + A(H+1, D+1)\} / 9$$

This example is directed to two dimensional weighted mean average filter applied to the first filter unit **25** as a simple filtering processing. Similarly, the phase information processing result **61** is also filtered by the two dimensional weighted mean average filter applied to the second filter unit **26,** and outputted to the boundary detecting unit **7.**

In this case, the first filter unit **25** performs the two dimensional weighted mean average of nine amplitude information processing results A(H-1, D-1), A(H-1, D), A(H-1, D+1), A(H, D-1), A(H, D), A(H, D+1), A(H+1, D-1), A(H+1, D), and A(H+1, D+1). The amplitude information processing result A(H, D) corresponds to a target point, while the amplitude information processing results A(H-1, D-1), A(H-1, D), A(H-1, D+1), A(H, D-1), A(H, D+1), A(H+1, D-1), A(H+1, D), and A(H+1, D+1) correspond to eight neighboring points. The second filter unit **26** performs the two dimensional weighted mean average of nine phase information processing results. However, the present invention is not limited to this case. For example, the number of data needed for the two dimensional weighted mean average is not limited. The first and second filter units **25** and **26** may be constituted by conventional two dimentional FIR filters, two dimentional non-linear filters, or the like. The first and second filter units **25** and **26** may be operable to remive noises from the amplitude information processing result **51** and the phase information processing result **61**. It is obvious that the filter applied to the amplitude information processing result **51** may be different in type or in characteristics from that applied to the phase information processing result **61**.

In the ultrasonograph according thus constructed, the boundary detecting unit **7** can obtain position information **71** indicative of the position of the boundary between the blood flow region and the intima and position information **72** indicative of the position of the boundary between the media and the adventitia without being easily affected from noises contained in the amplitude and phase information processing results obtained from the ultrasonic echo.

The position information **71** indicative of the position of the boundary between the blood flow region and the intima and the position information **72** indicative of the position of the boundary between the media and the adventitia obtained by the boundary detecting unit **7** are outputted to the third filter unit **27,** and filtered so that a longitudinal view of a blood vessel detected in a region of interst is displayed on a screen. The function of the third filter unit **27** is to mainly remove noises from the position information **71** indicative of the position of the boundary between the blood flow region and the intima and the position information **72** indicative of the position of the boundary between the media and the adventitia, to remove or reduce the significant deviation of the position of the the boundary of the blood vessel detected in a region of interest from a boundary visually recognized by an operator from luminance information based on an intensity of an ultrasonic echo, and to aboid or improve the unnatural and wrong results.

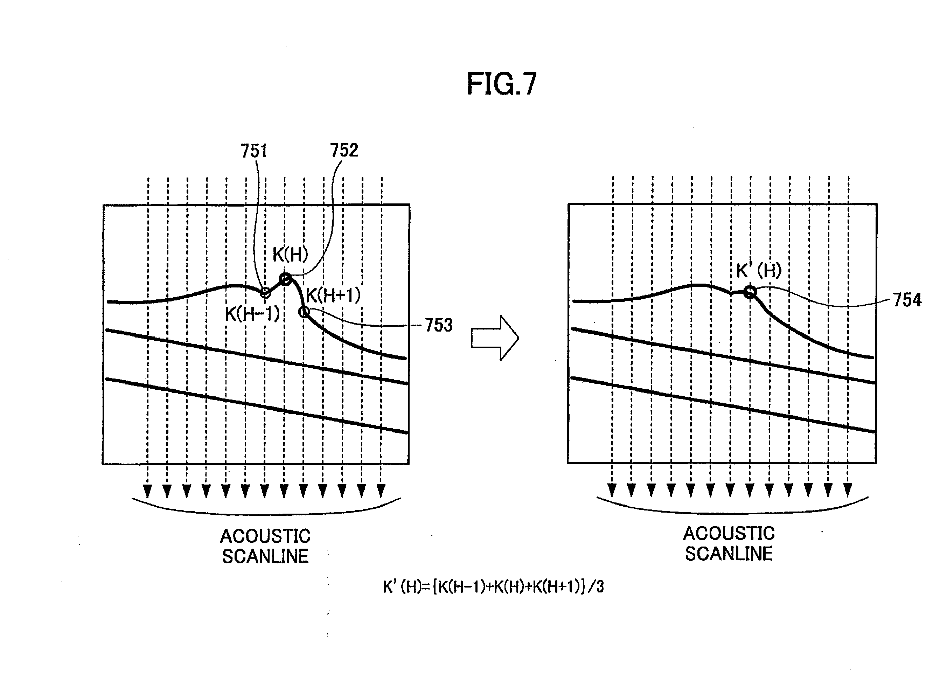

The following description is directed to an example of a filtering processing to be executed by the third filter unit 27. In FIG. 7, the position of the boundary K(H) is on an acoustic scanline H showing a path of an ultrasonic pulse signal. One of the filtered results outputted to the IMT value calculating unit 9 from the third filter unit 28 is represented by K'(H). The filtered result K'(H) is obtained through step of filtering the position information 71 indicative of the position of the boundary between the blood flow region and the intima, and expressed as the following formula.

$$K'(H) = \{K(H\text{-}1) + K(H) + K(H\text{+}1)\} / 3$$

This example is directed to the simplest weight mean filter applied to the third filter unit 27 in which the position information 71 indicative of the position of the boundary between the blood flow region and the intima is filterd. Similarly, the position information 72 indicative of the position of the boundary between the media and the adventitia is also filterd by this weight mean filter applied to the third filter unit 27.

As explained above, the ultrasonograph according to the second embodiment comprises an ultrasonic probe 101 for emitting at leass one ultrasonic pulse signal in a direction from a skin surface 20 of an object toward a blood vessel 30 inside the object, and receiving an ultrasonic echo from the blood vessel 30 as a reflection of the ultrasonic pulse signal from the blood vessel 30, a receiving unit 2 for converting the ultrasonic echo received by the ultrasonic probe 101 into an ultrasonic echo, an amplitude information processing unit 5 for processing amplitude information indicative of an amplitude of the ultrasonic echo coming from a direction intersecting a longitudinal axis of the blood vessel 30, a phase information processing unit 6 for processing phase information indicative of a phase of the ultrasonic echo coming from a direction intersecting a longitudinal axis of the blood vessel 30, a boundary detecting unit 7 for detecting, on the basis of at least one processing result outputted from the amplitude information processing unit 5 and at least one processing result outputted from the phase information processing unit 6, a boundary 310 between a blood flow region and an intima and a boundary 320 between a media and an adventitia 301, and an IMT value calculating unit 9 for calculating an IMT value from the position information 71 indicative of the position of the boundary 310 between the blood flow region and the intima and the position information 72 indicative of the position of the boundary 320 between the media and the adventitia 301 detected by the boundary detecting unit 7. The ultrasonograph according to the embodiment further comprises a first filter unit 25 for filtering the processing result of the amplitude information processing unit 5, and outputting the filtered result to the boundary detecting unit 7, a second filter unit 26 for filtering the processing result of the amplitude information processing unit 6, and outputting the filtered result to the boundary detecting unit 7, a third filter unit 27 for filtering the processing result of the boundary detecting unit 7, and outputting the filtered result to the IMT value calculating unit 9 and the image synthesizing unit 10, and a filter control unit 28 for controlling the first filter unit 25, the second filter unit 26, and the third filter unit 27. Therefore, the ultrasonograph according to the second embodiment can accurately detect a boundary between a blood flow region and a vascular wall, without being significantly affected by the change of a luminance value obtained as an intima of the vascular wall even when the blood vessel has a local pathology such as for example atheroma. In the ultrasonograph according to the second embodiment, the IMT value calculating unit 9 can accurately calculate an IMT value without being easily affected by noises contained in the position information 71 indicative of the position of the boundary between the blood flow region and the intima and noises contained in the position information 72 indicative of the position of the boundary between the media and the adventitia detected by the boundary detecting unit 7, by reason that noises contained in the position information 71 indicative of the position of the boundary between the blood flow region and the intima and noises contained in the position information 72 indicative of the position of the boundary between the media and the adventitia detected by the boundary detecting unit 7 are removed. The image synthesizing unit 10 can accurately combine the image information produced by the B-mode processing unit 4 with the image information produced by the boudanry deceting unit 7. Therefore, the positions of the detected boundaries are closely equal to those visually recognized from the luminance information obtained on the basis of the intensity of the ultrasonic echo, and do not recognized as unnatural and wrong results. Furthermore, the ultrasonograph according to the present invention can accurately measure an IMT value of a vascular wall of a blood vessel in a region of interest.

Additionally, the third filter unit 27 performs the weighted mean average of three processing results K(H-1), K(H), and K(H+1) as mentioned above. The processing result K(H) corresponds to a target point, while the processing results K(H-1) and K(H+1) correspond to two neighboring points. However, the present invention is not limited to this case. For example, the number of data needed for the weighted mean average is not limited. The third filter unit 27 may be constituted by a conventional FIR filter, a non-linear filter, or the like operable to remove noises contained in the position information 71 indicative of the position of the boundary between the blood flow region and the intima and noises contained in the position information 72 indicative of the position of the boundary between the media and the adventitia detected by the boundary detecting unit 7. It is obvious that the filter applied to the position information 71 indicative of the position of the boundary between the blood flow region and the intima may be different in type or in characteristics from that applied to the position information **72** indicative of the position of the boundary between the media and the adventitia. Further, the filter control unit **28** may set filter coefficients applied to the first filter unit **25,** the second filter unit **26** and the third filter unit **27,** by cooperating with another device or by an operator's manual controls.

Industrial Applicability of the Invention

As will be seen the foregoing description, the ultrasonograph according to the present invention can accurately detect the boundary between the blood flow region **304** and the vascular wall **305, 311** without being significantly affected by the change of a luminance value obtained as an intima even when the blood vessel has a local pathology such as atheroma. Further, the position of the boundary between the blood flow region **304** and the vascular wall **305, 311** and the position of the intima detected in the above-mentuioned method do not deviate significantly from these visually determined by an operator on the basis of luminance information obtained from the intensity of the ultrasonic echo, and do not recognized as unnatural and wrong results. Accordingly, the ultrasonograph according to the present invention can accurately measure the IMT value of the vascular wall by combining information obtained from the intensity B(D) of the ultrasonic echo with information extracted from characteristics of tissues, automatically detecting the position of the boundary between the blood flow region **304** and the vascular wall **305, 311** and the location of a media, which are significantly close to those determined visually by an operator, and is useful for diagnosing or displaying an image indicative of a condition of a blood vessel, by using an ultrasonic wave, in the medical field.

**Claims**

1. An ultrasonograph comprising:

   an ultrasonic signal emitting unit (101) for emitting at least one ultrasonic signal in a depth direction from a skin surface of an object toward a blood vessel (30) of said object;
   an ultrasonic echo transmitting unit (2) for receiving an ultrasonic echo from tissues of said object when said ultrasonic signal is emitted by said ultrasonic signal emitting unit (101), and converting said ultrasonic echo into an electric signal;
   an amplitude information processing unit (5) for processing amplitude information indicative of an amplitude of said ultrasonic echo along a direction intersecting a central axis of said blood vessel (30); and
   a boundary detecting unit (7) for detecting a position of a boundary between a blood flow region of said blood vessel (30) and an intima of said blood vessel (30) and a position of a boundary between a media of said blood vessel (30) and an adventitia (301) of said blood vessel (30),
   wherein said ultrasonograph further comprises
   a phase information processing unit (6) for processing phase information indicative of a phase of said ultrasonic echo along said direction intersecting said central axis of said blood vessel (30); and
   a region determining unit (8) for determining a region of interest (40) covering at least one of a front vascular wall which is a vascular wall close to said ultrasonic signal emitting unit (101) and a back vascular wall which is a vascular wall far from said ultrasonic signal emitting unit (101),
   **characterised by**
   said boundary detecting unit (7) detecting boundary positions on the basis of at least one processing result outputted from said amplitude information processing unit (5) and at least one processing result outputted from said phase information processing unit (6), said processing results outputted from said amplitude information processing unit (5) being an intensity of said ultrasonic echo coming from a depth direction of said object and a rate of change of said intensity in said depth direction from said skin surface, said processing result outputted from said phase information processing unit (6) being a hardness of said tissues of said object in said depth direction from said skin surface calculated from said phase information of said ultrasonic echo, said boundary detecting unit (7) determining a detection range within said region of interest (40) on the basis of said intensity of said ultrasonic echo, and detecting said position of a boundary between a blood flow region (304) of said blood vessel (30) and the intima of the blood vessel (30) within said detection range on the basis of said hardness of said tissues of said object, and detecting said position of a boundary between the media of said blood vessel (30) and the adventitia (301) of the blood vessel (30) within said detection range on the basis of said rate of change of said intensity in said depth direction from said skin surface.

2. An ultrasonograph according to claim 1, further comprising:

an IMT value calculating unit (9) for calculating an IMT value indicative of a thickness of a vascular wall defined by said intima and said media on the basis of position information indicative of said position of said boundary between said blood flow region (304) and said intima and position information indicative of said position of said boundary between said media and said adventitia (301) detected by said boundary detecting unit (7).

3. An ultrasonograph according to claim 1 or claim 2, wherein
said amplitude information processing unit (5) is adapted to process said amplitude information of said ultrasonic echo from said region of interest (40) determined by said region determining unit (8), and
said phase processing unit (6) is adapted to process said phase information of said ultrasonic echo from said region of interest (40) determined by said region determining unit (8).

4. An ultrasonograph according to any one of claims 1 to 3, wherein
said boundary detecting unit (7) is adapted to detect, on the basis of one heart cycle, said boundary between said blood flow region (304) and said intima and said boundary between said media and said adventitia (301) from said processing result outputted from said amplitude information processing unit (5) and said processing result outputted from said phase information processing unit (6).

5. An ultrasonograph according to any one of claims 1 to 4, wherein
one of said processing result outputted from said amplitude information processing unit (5) is a hardness of tissues of said object calculated in a depth direction of said object, from said phase information of said ultrasonic echo.

6. An ultrasonograph according to any one of claims 1 to 4, wherein
one of said processing result outputted from said amplitude information processing unit (5) is a strain of tissues of said object calculated, in a depth direction of said object, from said phase information of said ultrasonic echo on the basis of one heart cycle.

7. An ultrasonograph according to any one of claims 1 to 4, wherein
one of said processing result outputted from said amplitude information processing unit (5) is a thickness of tissues of said object calculated, in a depth direction of said object, from said phase information of said ultrasonic echo.

8. An ultrasonograph according to any one of claims 1 to 4, wherein
one of said processing result outputted from said amplitude information processing unit (5) is a moving velocity of tissues of said object calculated, in a depth direction of said object, from said phase information of said ultrasonic echo on the basis of one heart cycle.

9. An ultrasonograph according to any one of claims 1 to 8, wherein
said boundary detecting unit (7) is adapted to determine a region of detection in a depth direction of said object on the basis of at least one processing result outputted from said amplitude information processing unit (5), and to detect said boundary between said blood flow region (304) and said intima on the basis of at least one processing result outputted from said phase information processing unit (6) in said region of detection.

10. An ultrasonograph according to claim 9, wherein
said processing result outputted from said amplitude information processing unit (5) is an intensity of said ultrasonic echo, and
said processing result outputted from said phase information processing unit (6) is a hardness of tissues of said object calculated, in said depth direction of said object, from said phase information of said ultrasonic echo.

11. An ultrasonograph according to any one of claims 1 to 8, wherein
said boundary detecting unit (7) is adapted to determine a region of detection in a depth direction of said object on the basis of at least one processing result outputted from said amplitude information processing unit (5), and to detect said boundary between said media and said adventitia (301) on the basis of at least one processing result outputted from said phase information processing unit (6) in said region of detection.

12. An ultrasonograph according to claim 11, wherein
at least one processing result outputted from said amplitude information processing unit (5) is an intensity of an ultrasonic echo coming from a depth direction of said object and the rate of change of said intensity of said ultrasonic echo.

**13.** An ultrasonograph according to any one of claims 1 to 12, wherein
at least one processing result outputted from said amplitude information processing unit (5) is filtered in a depth direction of said object, and then outputted to said boundary detecting unit (7).

**14.** An ultrasonograph according to any one of claims 1 to 13, wherein
at least one processing result outputted from said amplitude information processing unit (5) is filtered in a depth direction of said object and in a longitudinal direction of said blood vessel (30), and then outputted to said boundary detecting unit (7).

**15.** An ultrasonograph according to any one of claims 1 to 14, wherein
at least one processing result outputted from said phase information processing unit (6) is filtered in a depth direction of said object, and then outputted to said boundary detecting unit (7).

**16.** An ultrasonograph according to any one of claims 1 to 15, wherein
at least one processing result outputted from said phase information processing unit (6) is filtered in a depth direction of said object and in a longitudinal direction of said blood vessel (30), and then outputted to said boundary detecting unit (7).

**17.** An ultrasonograph according to any one of claims 1 to 16, wherein
position information indicative of said position of said boundary between said blood flow region (304) and said intima and position information indicative of said position of said boundary between said media and said adventitia (301) detected by said boundary detecting unit (7) are filtered in a longitudinal direction of said blood vessel (30), and then outputted to said IMT value calculating unit (9).

**Patentansprüche**

**1.** Sonograph, aufweisend:

eine Ultraschallsignal-Emittierungseinheit (101) zum Emittieren von zumindest einem Ultraschallsignal in einer Tiefenrichtung von einer Hautoberfläche eines Objekts in Richtung eines Blutgefäßes (30) des Objekts;
eine Ultraschallecho-Übertragungseinheit (2) zum Empfangen eines Ultraschallechos von Geweben des Objekts, wenn das Ultraschallsignal durch die Ultraschallsignal-Emittierungseinheit (101) emittiert wird, und Umwandeln des Ultraschallechos in ein elektrisches Signal;
eine Amplitudeninformations-Verarbeitungseinheit (5) zum Verarbeiten von Amplitudeninformationen, die eine Amplitude des Ultraschallechos entlang einer Richtung anzeigen, welche eine zentrale Achse des Blutgefäßes (30) kreuzt; und
eine Grenzerfassungseinheit (7) zum Erfassen einer Position einer Grenze zwischen einem Blutflussgebiet des Blutgefäßes (30) und einer Intima des Blutgefäßes (30) sowie einer Position einer Grenze zwischen einer Media des Blutgefäßes (30) und einer Adventitia (301) des Blutgefäßes (30),
wobei der Sonograph ferner aufweist:

eine Phaseninformations-Verarbeitungseinheit (6) zum Verarbeiten von Phaseninformationen, die eine Phase des Ultraschallechos entlang der Richtung anzeigen, welche die zentrale Achse des Blutgefäßes (30) kreuzt; und
eine Gebietsbestimmungseinheit (8) zum Bestimmen eines Gebiets von Interesse (40), das zumindest eine einer vorderen Gefäßwand, welche eine Gefäßwand in der Nähe der Ultraschallsignal-Emittierungseinheit (101) ist, und einer hinteren Gefäßwand bedeckt, welche eine Gefäßwand weit von der Ultraschallsignal-Emittierungseinheit (101) entfernt ist,
**dadurch gekennzeichnet, dass**
die Grenzerfassungseinheit (7) Grenzpositionen auf der Basis von zumindest einem von der Amplitudeninformations-Verarbeitungseinheit (5) ausgegebenen Verarbeitungsergebnis und zumindest einem von der Phaseninformations-Verarbeitungseinheit (6) ausgegebenen Verarbeitungsergebnis erfasst, wobei die von der Amplitudeninformations-Verarbeitungseinheit (5) ausgegebenen Verarbeitungsergebnisse eine Intensität des aus einer Tiefenrichtung des Objekts kommenden Ultraschallechos und eine Änderungsrate der Intensität in der Tiefenrichtung von der Hautoberfläche darstellen, wobei das von der Phaseninformations-Verarbeitungseinheit (6) ausgegebene Verarbeitungsergebnis eine Härte der Geweben des Objekts in der Tiefenrichtung von der Hautoberfläche darstellt, das aus den Phaseninformationen des Ultraschallechos berechnet ist, wobei die Grenzerfassungseinheit (7) einen Erfassungsbereich innerhalb des Gebiets von Interesse (40) auf der Basis der Intensität des Ultraschallechos bestimmt und die Position einer Grenze zwischen einem Blutflussgebiet (304) des Blutgefäßes (30) und der Intima des Blutgefäßes (30) innerhalb des Erfassungsbereichs auf der Basis der Härte der Geweben des Objekts erfasst und die Position einer Grenze zwischen der Media des Blutgefäßes (30) und der Adventitia (301) des Blutgefäßes (30) innerhalb des Erfassungsbereichs auf der Basis der Änderungsrate der Intensität in der Tiefenrichtung von der Hautoberfläche erfasst.

2. Sonograph nach Anspruch 1, ferner aufweisend:

   eine IMT-Wert-Berechnungseinheit (9) zum Berechnen eines IMT-Werts, der eine Dicke einer durch die Intima und die Media definierten Gefäßwand anzeigt, auf der Basis von Positionsinformationen, welche die Position der Grenze zwischen dem Blutflussgebiet (304) und der Intima anzeigen, und Positionsinformationen, welche die Position der durch die Grenzerfassungseinheit (7) erfassten Grenze zwischen der Media und der Adventitia (301) anzeigen.

3. Sonograph nach Anspruch 1 oder 2, wobei
   die Amplitudeninformations-Verarbeitungseinheit (5) angepasst ist, um die Amplitudeninformationen des Ultraschallechos von dem durch die Gebietsbestimmungseinheit (8) bestimmten Gebiet von Interesse (40) zu verarbeiten, und
   die Phasenverarbeitungseinheit (6) angepasst ist, um die Phaseninformationen des Ultraschallechos von dem durch die Gebietsbestimmungseinheit (8) bestimmten Gebiet von Interesse (40) zu verarbeiten.

4. Sonograph nach einem der Ansprüche 1 bis 3, wobei
   die Grenzerfassungseinheit (7) angepasst ist, um auf der Basis eines Herzzyklus die Grenze zwischen dem Blutflussgebiet (304) und der Intima sowie die Grenze zwischen der Media und der Adventitia (301) aus dem von der Amplitudeninformations-Verarbeitungseinheit (5) ausgegebenen Verarbeitungsergebnis sowie dem von der Phaseninformations-Verarbeitungseinheit (6) ausgegebenen Verarbeitungsergebnis zu erfassen.

5. Sonograph nach einem der Ansprüche 1 bis 4, wobei
   eines des von der Amplitudeninformations-Verarbeitungseinheit (5) ausgegebenen Verarbeitungsergebnisses eine in einer Tiefenrichtung des Objekts aus den Phaseninformationen des Ultraschallechos berechnete Härte von Geweben des Objekts darstellt.

6. Sonograph nach einem der Ansprüche 1 bis 4, wobei
   eines des von der Amplitudeninformations-Verarbeitungseinheit (5) ausgegebenen Verarbeitungsergebnisses eine in einer Tiefenrichtung des Objekts aus den Phaseninformationen des Ultraschallechos auf der Basis eines Herzzyklus berechnete Belastung von Geweben des Objekts darstellt.

7. Sonograph nach einem der Ansprüche 1 bis 4, wobei
   eines des von der Amplitudeninformations-Verarbeitungseinheit (5) ausgegebenen Verarbeitungsergebnisses eine in einer Tiefenrichtung des Objekts aus den Phaseninformationen des Ultraschallechos berechnete Dicke von Geweben des Objekts darstellt.

8. Sonograph nach einem der Ansprüche 1 bis 4, wobei
   eines des von der Amplitudeninformations-Verarbeitungseinheit (5) ausgegebenen Verarbeitungsergebnisses eine in einer Tiefenrichtung des Objekts aus den Phaseninformationen des Ultraschallechos auf der Basis eines Herzzyklus berechnete Bewegungsgeschwindigkeit von Geweben des Objekts darstellt.

9. Sonograph nach einem der Ansprüche 1 bis 8, wobei
   die Grenzerfassungseinheit (7) angepasst ist, um ein Erfassungsgebiet in einer Tiefenrichtung des Objekts auf der Basis von zumindest einem von der Amplitudeninformations-Verarbeitungseinheit (5) ausgegebenen Verarbeitungsergebnis zu bestimmen, und um die Grenze zwischen dem Blutflussgebiet (304) und der Intima auf der Basis von zumindest einem von der Phaseninformations-Verarbeitungseinheit (6) in dem Erfassungsgebiet ausgegebenen Verarbeitungsergebnis zu erfassen.

10. Sonograph nach Anspruch 9, wobei
    das von der Amplitudeninformations-Verarbeitungseinheit (5) ausgegebene Verarbeitungsergebnis eine Intensität des Ultraschallechos darstellt, und

das von der Phaseninformations-Verarbeitungseinheit (6) ausgegebene Verarbeitungsergebnis eine in der Tiefenrichtung des Objekts aus den Phaseninformationen des Ultraschallechos berechnete Härte von Geweben des Objekts darstellt.

11. Sonograph nach einem der Ansprüche 1 bis 8, wobei
die Grenzerfassungseinheit (7) angepasst ist, um ein Erfassungsgebiet in einer Tiefenrichtung des Objekts auf der Basis von zumindest einem von der Amplitudeninformations-Verarbeitungseinheit (5) ausgegebenen Verarbeitungsergebnis zu bestimmen, und um die Grenze zwischen der Media und der Adventitia (301) auf der Basis von zumindest einem von der Phaseninformations-Verarbeitungseinheit (6) in dem Erfassungsgebiet ausgegebenen Verarbeitungsergebnis zu erfassen.

12. Sonograph nach Anspruch 11, wobei
zumindest ein von der Amplitudeninformations-Verarbeitungseinheit (5) ausgegebenes Verarbeitungsergebnis eine Intensität eines aus einer Tiefenrichtung des Objekts kommenden Ultraschallechos und die Änderungsrate der Intensität des Ultraschallechos darstellt.

13. Sonograph nach einem der Ansprüche 1 bis 12, wobei
zumindest ein von der Amplitudeninformations-Verarbeitungseinheit (5) ausgegebenes Verarbeitungsergebnis in einer Tiefenrichtung des Objekts gefiltert und dann an die Grenzerfassungseinheit (7) ausgegeben wird.

14. Sonograph nach einem der Ansprüche 1 bis 13, wobei
zumindest ein von der Amplitudeninformations-Verarbeitungseinheit (5) ausgegebenes Verarbeitungsergebnis in einer Tiefenrichtung des Objekts und in einer longitudinalen Richtung des Blutgefäßes (30) gefiltert und dann an die Grenzerfassungseinheit (7) ausgegeben wird.

15. Sonograph nach einem der Ansprüche 1 bis 14, wobei
zumindest ein von der Phaseninformations-Verarbeitungseinheit (6) ausgegebenes Verarbeitungsergebnis in einer Tiefenrichtung des Objekts gefiltert und dann an die Grenzerfassungseinheit (7) ausgegeben wird.

16. Sonograph nach einem der Ansprüche 1 bis 15, wobei
zumindest ein von der Phaseninformations-Verarbeitungseinheit (6) ausgegebenes Verarbeitungsergebnis in einer Tiefenrichtung des Objekts und in einer longitudinalen Richtung des Blutgefäßes (4) gefiltert und dann an die Grenzerfassungseinheit (7) ausgegeben wird.

17. Sonograph nach einem der Ansprüche 1 bis 16, wobei
Positionsinformationen, welche die Position der Grenze zwischen dem Blutflussgebiet (304) und der Intima anzeigen, und Positionsinformationen, welche die Position der Grenze zwischen der Media und der Adventitia (301) anzeigen, die durch die Grenzerfassungseinheit (7) erfasst sind, werden in einer longitudinalen Richtung des Blutgefäßes (30) gefiltert und dann an die IMT-Wert-Berechnungseinheit (9) ausgegeben.

**Revendications**

1. Echographe comprenant :

une unité d'émission de signal ultrasonore (101) pour émettre au moins un signal ultrasonore dans une direction de profondeur à partir d'une surface de peau d'un objet vers un vaisseau sanguin (30) dudit objet ;
une unité de transmission d'écho ultrasonore (2) pour recevoir un écho ultrasonore provenant de tissus dudit objet quand ledit signal ultrasonore est émis par ladite unité d'émission de signal ultrasonore (101) et convertir ledit écho ultrasonore en un signal électrique ;
une unité de traitement d'informations d'amplitude (5) pour traiter des informations d'amplitude indicatives d'une amplitude dudit écho ultrasonore le long d'une direction coupant un axe central dudit vaisseau sanguin (30) ; et
une unité de détection de limite (7) pour détecter une position d'une limite entre une région d'écoulement sanguin dudit vaisseau sanguin (30) et une intima dudit vaisseau sanguin (30) et une position d'une limite entre une media dudit vaisseau sanguin (30) et une adventice (301) dudit vaisseau sanguin (30),
dans lequel ledit échographe comprend en outre
une unité de traitement d'informations de phase (6) pour traiter des informations de phase indicatives d'une phase dudit écho ultrasonore le long de ladite direction coupant ledit axe central dudit vaisseau sanguin (30) ; et

une unité de détermination de région (8) pour déterminer une région d'intérêt (40) couvrant au moins une parmi une paroi vasculaire avant qui est une paroi vasculaire proche de ladite unité d'émission de signal ultrasonore (101) et une paroi vasculaire arrière qui est une paroi vasculaire éloignée de ladite unité d'émission de signal ultrasonore (101),

**caractérisé en ce que**
ladite unité de détection de limite (7) détecte des positions de limite sur la base d'au moins un résultat de traitement délivré en sortie par ladite unité de traitement d'informations d'amplitude (5) et d'au moins un résultat de traitement délivré en sortie par ladite unité de traitement d'informations de phase (6), lesdits résultats de traitement délivrés en sortie par ladite unité de traitement d'informations d'amplitude (5) étant une intensité dudit écho ultrasonore venant d'une direction de profondeur dudit objet et un taux de variation de ladite intensité dans ladite direction de profondeur à partir de ladite surface de peau, ledit résultat de traitement délivré en sortie par ladite unité de traitement d'informations de phase (6) étant une dureté desdits tissus dudit objet dans ladite direction de profondeur à partir de ladite surface de peau calculée à partir desdites informations de phase dudit écho ultrasonore, ladite unité de détection de limite (7) déterminant une plage de détection à l'intérieur de ladite région d'intérêt (40) sur la base de ladite intensité dudit écho ultrasonore et détectant ladite position d'une limite entre une région d'écoulement sanguin (304) dudit vaisseau sanguin (30) et l'intima du vaisseau sanguin (30) dans ladite plage de détection sur la base de ladite dureté desdits tissus dudit objet et détectant ladite position d'une limite entre la media dudit vaisseau sanguin (30) et l'adventice (301) du vaisseau sanguin (30) dans ladite plage de détection sur la base dudit taux de variation de ladite intensité dans ladite direction de profondeur à partir de ladite surface de peau.

2. Echographe selon la revendication 1, comprenant en outre :

une unité de calcul de valeur de EIM (9) pour calculer une valeur de EIM indicative d'une épaisseur d'une paroi vasculaire définie par ladite intima et ladite media sur la base d'informations de position indicatives de ladite position de ladite limite entre ladite région d'écoulement sanguin (304) et ladite intima et d'informations de position indicatives de ladite position de ladite limite entre ladite media et ladite adventice (301) détectée par ladite unité de détection de limite (7).

3. Echographe selon la revendication 1 ou la revendication 2, dans lequel
ladite unité de traitement d'informations d'amplitude (5) est adaptée pour traiter lesdites informations d'amplitude dudit écho ultrasonore provenant de ladite région d'intérêt (40) déterminée par ladite unité de détermination de région (8), et
ladite unité de traitement de phase (6) est adaptée pour traiter lesdites informations de phase dudit écho ultrasonore provenant de ladite région d'intérêt (40) déterminée par ladite unité de détermination de région (8).

4. Echographe selon l'une quelconque des revendications 1 à 3, dans lequel
ladite unité de détection de limite (7) est adaptée pour détecter, sur la base d'un cycle cardiaque, ladite limite entre ladite région d'écoulement sanguin (304) et ladite intima et ladite limite entre ladite media et ladite adventice (301) à partir dudit résultat de traitement délivré en sortie par ladite unité de traitement d'informations d'amplitude (5) et dudit résultat de traitement délivré en sortie par ladite unité de traitement d'informations de phase (6).

5. Echographe selon l'une quelconque des revendications 1 à 4, dans lequel
un dudit résultat de traitement délivré en sortie par ladite unité de traitement d'informations d'amplitude (5) est une dureté de tissus dudit objet calculée, dans une direction de profondeur dudit objet, à partir desdites informations de phase dudit écho ultrasonore.

6. Echographe selon l'une quelconque des revendications 1 à 4, dans lequel
un dudit résultat de traitement délivré en sortie par ladite unité de traitement d'informations d'amplitude (5) est une déformation de tissus dudit objet calculée, dans une direction de profondeur dudit objet, à partir desdites informations de phase dudit écho ultrasonore sur la base d'un cycle cardiaque.

7. Echographe selon l'une quelconque des revendications 1 à 4, dans lequel
un dudit résultat de traitement délivré en sortie par ladite unité de traitement d'informations d'amplitude (5) est une épaisseur de tissus dudit objet calculée, dans une direction de profondeur dudit objet, à partir desdites informations de phase dudit écho ultrasonore.

8. Echographe selon l'une quelconque des revendications 1 à 4, dans lequel

un dudit résultat de traitement délivré en sortie par ladite unité de traitement d'informations d'amplitude (5) est une vitesse de déplacement de tissus dudit objet calculée, dans une direction de profondeur dudit objet, à partir desdites informations de phase dudit écho ultrasonore sur la base d'un cycle cardiaque.

9.  Echographe selon l'une quelconque des revendications 1 à 8, dans lequel
ladite unité de détection de limite (7) est adaptée pour déterminer une région de détection dans une direction de profondeur dudit objet sur la base d'au moins un résultat de traitement délivré en sortie par ladite unité de traitement d'informations d'amplitude (5) et pour détecter ladite limite entre ladite région d'écoulement sanguin (304) et ladite intima sur la base d'au moins un résultat de traitement délivré en sortie par ladite unité de traitement d'informations de phase (6) dans ladite région de détection.

10. Echographe selon la revendication 9, dans lequel
ledit résultat de traitement délivré en sortie par ladite unité de traitement d'informations d'amplitude (5) est une intensité dudit écho ultrasonore, et
ledit résultat de traitement délivré en sortie par ladite unité de traitement d'informations de phase (6) est une dureté de tissus dudit objet calculée, dans ladite direction de profondeur dudit objet, à partir desdites informations de phase dudit écho ultrasonore.

11. Echographe selon l'une quelconque des revendications 1 à 8, dans lequel
ladite unité de détection de limite (7) est adaptée pour déterminer une région de détection dans une direction de profondeur dudit objet sur la base d'au moins un résultat de traitement délivré en sortie par ladite unité de traitement d'informations d'amplitude (5) et pour détecter ladite limite entre ladite media et ladite adventice (301) sur la base d'au moins un résultat de traitement délivré en sortie par ladite unité de traitement d'informations de phase (6) dans ladite région de détection.

12. Echographe selon la revendication 11, dans lequel
au moins un résultat de traitement délivré en sortie par ladite unité de traitement d'informations d'amplitude (5) est une intensité d'un écho ultrasonore venant d'une direction de profondeur dudit objet et le taux de variation de ladite intensité dudit écho ultrasonore.

13. Echographe selon l'une quelconque des revendications 1 à 12, dans lequel
au moins un résultat de traitement délivré en sortie par ladite unité de traitement d'informations d'amplitude (5) est filtré dans une direction de profondeur dudit objet et ensuite délivré en sortie à ladite unité de détection de limite (7).

14. Echographe selon l'une quelconque des revendications 1 à 13, dans lequel
au moins un résultat de traitement délivré en sortie par ladite unité de traitement d'informations d'amplitude (5) est filtré dans une direction de profondeur dudit objet et dans une direction longitudinale dudit vaisseau sanguin (30) et ensuite délivré en sortie à ladite unité de détection de limite (7).

15. Echographe selon l'une quelconque des revendications 1 à 14, dans lequel
au moins un résultat de traitement délivré en sortie par ladite unité de traitement d'informations de phase (6) est filtré dans une direction de profondeur dudit objet et ensuite délivré en sortie à ladite unité de détection de limite (7).

16. Echographe selon l'une quelconque des revendications 1 à 15, dans lequel
au moins un résultat de traitement délivré en sortie par ladite unité de traitement d'informations de phase (6) est filtré dans une direction de profondeur dudit objet et dans une direction longitudinale dudit vaisseau sanguin (30) et ensuite délivré en sortie à ladite unité de détection de limite (7).

17. Echographe selon l'une quelconque des revendications 1 à 16, dans lequel
des informations de position indicatives de ladite position de ladite limite entre ladite région d'écoulement sanguin (304) et ladite intima et des informations de position indicatives de ladite position de ladite limite entre ladite media et ladite adventice (301) détectée par ladite unité de détection de limite (7) sont filtrées dans une direction longitudinale dudit vaisseau sanguin (30) et ensuite délivrées en sortie à ladite unité de calcul de valeur de EIM (9).

# FIG.1

BOUNDARY BETWEEN BLOOD FLOW REGION AND INTIMA

BOUNDARY BETWEEN MEDIA AND ADVENTITIA

AMPLITUDE INFORMATION PROCESSING RESULT

PHASE INFORMATION PROCESSING RESULT

THRESHOLD SETTING

OFFSET SETTING

**FIG.2**

# FIG.3

(a)    (b)    (c)    (d)

RATE OF CHANGE OF
ECHO STRENGTH
IN DEPTH DIRECTION
dB(D)/dD

ECHO STRENGTH
B(D)

HARDNESS
VALUE
E(D)

THRESHOLD

Z1    F0    E0    P1

121    P2    F1    120

R0    R1    R2    R3    R4    R5

301    305    302    20    30    304    310    302    306    310    311    40    320    E5

DEPTH D    DEPTH D    73    DEPTH D

EP 1 997 436 B1

# FIG.4

EP 1 997 436 B1

# FIG.5

EP 1 997 436 B1

ACOUSTIC
SCANLINE

ACOUSTIC
SCANLINE

A(H-1,D-1)　A(H,D-1)　A(H+1,D-1)

A(H-1,D)　A(H,D)　A(H+1,D)

A(H-1,D+1)　A(H,D+1)　A(H+1,D+1)

A' (H,D)

$$A'(H,D) = \{A(H,D-1)+A(H,D)+A(H,D+1)\}/9$$

# FIG.6

$$A'(H,D)=[A(H-1,D-1)+A(H-1,D)+A(H-1,D+1)+A(H,D-1)+A(H,D)+A(H,D+1)+A(H+1,D-1)+A(H+1,D)+A(H+1,D+1)]/9$$

EP 1 997 436 B1

# FIG.7

ACOUSTIC
SCANLINE

ACOUSTIC
SCANLINE

$$K'(H) = [K(H-1) + K(H) + K(H+1)] / 3$$

EP 1 997 436 B1

**EP 1 997 436 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H11318896 B

- JP 2004112568 A